# EUROPEAN PATENT APPLICATION

(11) **EP 1 541 581 A1**
(43) Date of publication of application: **15.06.2005**
(21) Application number: 03792774.6
(22) Date of filing: 21.08.2003
(51) Int. Cl.: C07H 19/067, C07H 19/073, C07H 19/167, C07H 19/173, A61K 31/7068, A61K 31/7072, A61K 31/7076, A61K 31/708, A61P 43/00

(54) **4 -THIONUCLEOTIDE**

(30) Priority: 22.08.2002 JP 2002242259
(71) Applicant: Geneticlab Co., Ltd., Sapporo-shi, Hokkaido 060-0009 (JP); Matsuda, Akira, Sapporo-shi, Hokkaido 060-0812 (JP)
(72) Inventor: MATSUDA, Akira, Medicinal Chemistry, Sapporo-shi, Hokkaido 060-0812 (JP); KATO, Yuka, Medicinal Chemistry, Sapporo-shi, Hokkaido 060-0812 (JP); MINAKAWA, Noriaki, Medicinal Chemistry, Sapporo-shi, Hokkaido 060-0812 (JP)
(74) Representative: VOSSIUS & PARTNER
(86) International application number: PCT/JP2003/010576
(87) International publication number: WO 2004/018494

(57) **Abstract**

Disclosed is a compound of formula I: 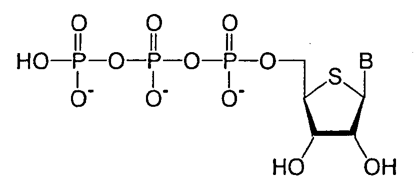 [wherein B is a nucleobase selected from the group consisting of adenine, guanine, cytosine, uracil and hypoxanthine], a compound of formula II: 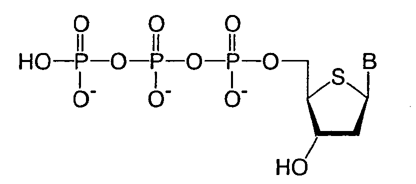 [wherein B' is a nucleobase selected from the group consisting of adenine, guanine, cytosine, thymine, uracil and hypoxanthine],
a method for synthesizing these nucleoside triphosphates, and a process for producing an oligonucleotide using these nucleoside triphosphates.

## Description

### Technical Field

The present invention relates to nucleotide analogues and a process for producing the same. More specifically, the invention relates to a 4'-thioribonucleotide and a 4'-thio-2'-deoxynucleotide, a process for producing these nucleotide analogues, and a process for producing oligonucleotides using these nucleotide analogues.

### Background Art

4'-Thionucleoside is a general term of nucleosides in which an oxygen atom of a furanose ring is substituted with a sulfur atom.

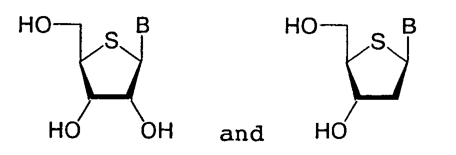

RNA or DNA containing a 4'-thioribonucleoside or a 4'-thio-2'-deoxyribonucleoside is suggested to be useful as an investigation reagent and a diagnostic or therapeutic agent, because it shows resistance to various nucleases.

Bellon et al. (Biochem. Biophys. Res. Comm., 1992, 184, 797-803) describe synthesis of oligodeoxynucleotides containing 1-(4-thio-β-D-ribofuranosyl)thymine. Bellon et al. (Nucleic Acids Res., 1993, 21, 1587-1593), Leydier et al. (Antisense Res. Rev. 1995, 5, 167-174) and Leydier et al. (Nucleosides Nucleotides, 1995, 14, 1027-1030) describe that a 4'-thio-β-D-oligoribonucleotide has high nuclease resistance. Dukhan et al. (Nucleosides Nucleotides, 1999, 18 1423-1424) describe synthesis of oligonucleotides containing four types of 4'-thioribonucleosides.

Bellon et al. (Nucleic Acids Res., 1993, 21, 1587-1593) have examined resistance to a degradation enzyme of RNA solely comprising 4'-thiouridine, and have reported that 4'-^{s}U₆ shows by far higher resistance to various degradation enzymes than native-type U₆.

Meanwhile, several synthesis examples of deoxyribonucleosides have been reported. Hancox et al. (Nucleic Acids Res., 1993, 21, 3485-3491) describe synthesis of 4'-thio-2'-deoxythimidine and oligodeoxyribonucleotide containing the same. Boggon et al. (Nucleic Acids Res., 1996, 24, 951-961) describe a structure of a synthetic DNA oligomer containing 4'-thio-2'-deoxythimidine. Jones et al. (Nucleic Acids Res., 1996, 24, 4117-4122) and Jones et al. (Bioorg. Med. Chem. Lett., 1997, 7, 1275-1278) describe a synthetic DNA oligomer containing 4'-thio-2'-deoxynucleotide. Kumar et al. (Nucleic Acids Res., 1997, 25, 2773-2783) describe a synthetic DNA oligomer containing 4'-thio-2'-deoxycytidine.

However, all of these oligoribonucleotides and oligodeoxyribonucleotides are produced by chemical synthesis. By this method, long-chain oligonucleotides can hardly be obtained, and the cost is high.

For synthesizing oligonucleotides from 4'-thionucleosides using an enzyme such as RNA polymerase, DNA polymerase or reverse transcriptase, 4-thionucleosides have to be triphosphorylated. Alexandrova et al. (Antiviral Chemistry & Chemotherapy, 1995, 7, 237-242) describe synthesis of 4'-thio-5-ethyl-2'-deoxyuridine 5'-triphosphate and recognition of this compound by DNA synthetase. However, 4'-thiodeoxyribonucleoside triphosphate or 4'-thioribonucleoside triphosphate with other nucleobase has not been obtained so far. This is presumably because stereoselective synthesis is difficult.

Thus, a novel improved method for synthesizing 4'-thionucleotides which can be recognized and elongated by an enzyme such as RNA polymerase or DNA polymerase is needed in the art.

Accordingly, the invention aims to provide novel nucleoside triphosphates, a method for synthesizing the same, and a process for producing oligonucleotides using these nucleoside triphosphates.

### Disclosure of the Invention

The invention provides a 4'-thioribonucleotide represented by formula I: 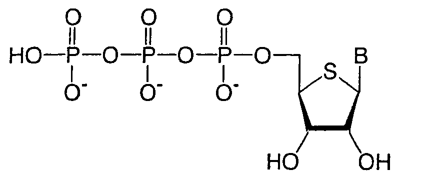 [wherein B is a nucleobase selected from the group consisting of adenine, guanine, cytosine, uracil and hypoxanthine].

The invention also provides a 4'-thio-2'-deoxynucleotide represented by formula II: 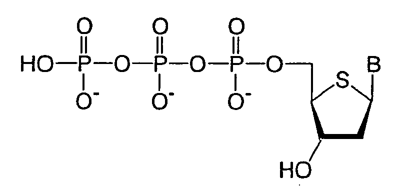 [wherein B' is a nucleobase selected from the group consisting of adenine, guanine, cytosine, thymine, uracil and hypoxanthine].

Since 4'-thionucleotides of the invention can be recognized and elongated with an enzyme such as RNA polymerase or DNA polymerase, they are useful as a monomer unit for synthesizing RNA or DNA having resistance to nucleases.

In another aspect, the invention provides a method for synthesizing a 4'-thioribonucleotide represented by formula I: 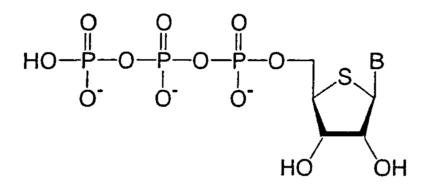 [wherein B is a nucleobase selected from the group consisting of adenine, guanine, cytosine, uracil and hypoxanthine] comprising reacting a compound of formula III: 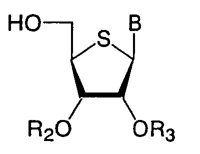 [wherein B is a nucleobase selected from the group consisting of adenine, guanine, cytosine, uracil and hypoxanthine, and each of R₂ and R₃ is independently a protecting group of a hydroxyl group]
with a compound of formula IV: 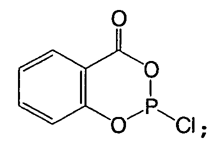 reacting the resulting intermediate with pyrophosphoric acid; and
conducting iodo-oxidation, hydrolysis and deprotection.

In still another aspect, the invention provides a method for synthesizing a 4'-thio-2'-deoxynucleotide represented by formula II: 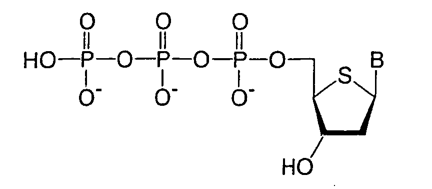 [wherein B is a nucleobase selected from the group consisting of adenine, guanine, cytosine, thymine, uracil and hypoxanthine]
comprising reacting a compound of formula V: 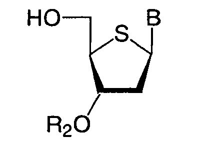 [wherein B is a nucleobase selected from the group consisting of adenine, guanine, cytosine, thymine, uracil and hypoxanthine, and R₂ is a protecting group of a hydroxyl group] with a compound of formula IV: 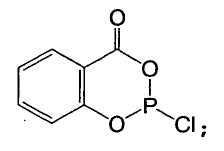 reacting the resulting intermediate with pyrophosphoric acid; and
conducting iodo-oxidation, hydrolysis and deprotection.

In another aspect of the invention, the invention provides a process for producing an oligonucleotide containing at least one nucleoside unit of formula VI: 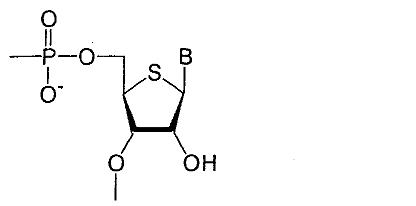 [wherein B is a nucleobase selected from the group consisting of adenine, guanine, cytosine, uracil and hypoxanthine] comprising conducting an RNA chain elongation reaction with RNA synthetase in the presence of the 4'-thioribonucleotide of the invention. The RNA synthetase includes RNA polymerase.

In still another aspect of the invention, the invention provides a process for producing an oligonucleotide containing at least one nucleotide unit of formula VII: 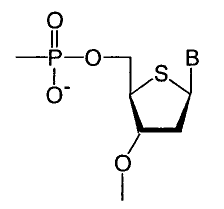 [wherein B is a nucleobase selected from the group consisting of adenine, guanine, cytosine, thymine, uracil and hypoxanthine]
comprising conducting a DNA chain reaction with DNA synthetase in the presence of the 4'-thio-2'-deoxynucleotide of the invention. Examples of the DNA synthetase include DNA polymerase, reverse transcriptase and terminal deoxynucleotide transferase.

Since RNA or DNA containing a 4'-thioribonucleoside or a 4'-thio-2'-deoxyribonucleoside produced by the process of the invention shows resistance to various nucleases, it is useful as an investigation reagent and as a diagnostic or therapeutic agent. According to the invention, since an oligonucleotide can be synthesized by means of an enzyme, an oligonucleotide having longer chain can easily be produced in comparison to the conventional chemical synthesis methods. Further, since RNA containing a 4'-thioribonucleoside produced by the process of the invention is recognized by reverse transcriptase, cDNA can be synthesized using this RNA as a template. Accordingly, it is quite useful in in-vitro selection.

### Brief Description of the Drawings

Fig. 1 shows a synthesis scheme of 4'-thio UTP described in Example 9.
Fig. 2 shows a synthesis scheme of 4'-thio CTP described in Example 10.
Fig. 3 shows a synthesis scheme of 4'-thio ATP described in Example 11.
Fig. 4 shows a synthesis scheme of 4'-thio GTP described in Example 12.
Fig. 5 shows a synthesis scheme of 4'-thio-2'-deoxy UTP described in Example 13.
Fig. 6 shows synthesis schemes of 4'-thio-2'-deoxy CTP, 4'-thio-2'-deoxy ATP and 4'-thio-2'-deoxy GTP described in Example 14.
Fig. 7 shows an outline of the experiment of incorporating 4'-thio UTP using T7 RNA polymerase.
Fig. 8 shows results of the experiment of incorporating 4'-thio UTP using T7 RNA polymerase.
Fig. 9 shows an outline of the experiment of incorporating 4'-thio UTP and 4'-thio CTP using T7 RNA polymerase.
Fig. 10 shows results of the experiment of incorporating 4'-thio UTP and 4'-thio CTP using T7 RNA polymerase.
Fig. 11 shows an outline of cDNA synthesis experiment from RNA containing 4'-thio UTP and 4'-thio CTP using reverse transcriptase.
Fig. 12 shows results of cDNA synthesis experiment from RNA containing 4'-thio UTP and 4'-thio CTP with reverse transcriptase.
Fig. 13 shows RNase resistance of RNA containing 4'-thio UTP and 4'-thio CTP.

### Detailed Description of the Invention

The nucleoside triphosphate of the invention can be produced by starting from known 4-thio sugar, stereoselectively introducing a nucleobase into the sugar and then selectively introducing a phosphate group at the 5'-position.

4'-Thiouridine can be obtained by appropriately protecting hydroxyl groups at the 2-, 3- and 5-positions of a 4-thio sugar 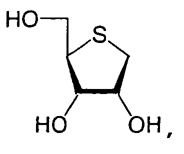 and stereoselectively introducing a nucleobase using a Pummerer reaction. 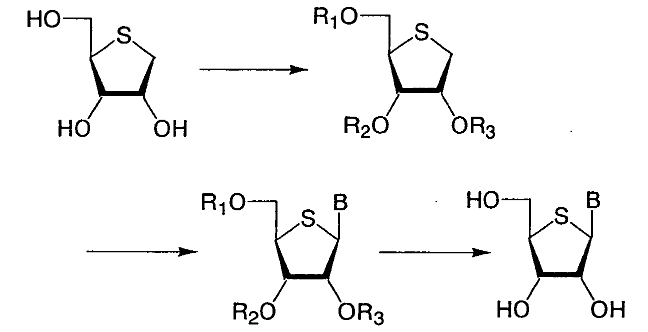 [wherein each of R₁, R₂ and R₃ independently represents a hydroxyl protecting group, R₁ and R₂ or R₂ and R₃ may together be a bifunctional hydroxyl protecting group].

When R₃ is an acyl protecting group having an electron-donating substituent, stereoselectivity is improved. It is preferably a 2,4-dimethoxybenzoyl group.

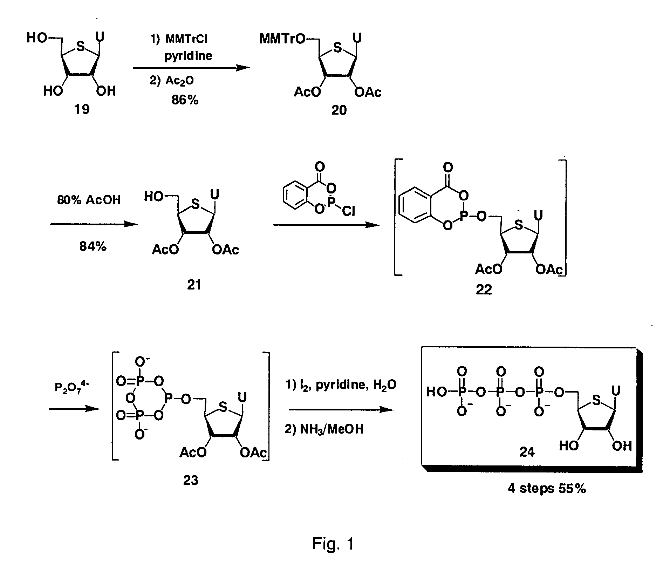

Uridine derivative 18 obtained by the Pummerer reaction can be converted to 4'-thiouridine 19 by conducting deprotection using ammonium fluoride and methylamine. Then, 4'-thio UTP is synthesized from 4'-thiouridine 19 (Fig. 1).

The 5'-position of compound 19 is monomethoxytritylated, and the 2'- and 3'-positions thereof are acetylated to obtain compound 20. Subsequently, demonomethoxytritylation is conducted to obtain acetyl compound 21. From the resulting compound 21, 4'-thio UTP 24 is synthesized by the method of Eckstein et al. (Luding, J. and Eckstein, F. (1989) J. Org. Chem., 54, 631-635). That is, the compound is converted into intermediate 22 using salicyl phosphorochloridite, and this intermediate is treated with pyrophosphoric acid to form a cyclotriphosphite compound 23. Iodo-oxidation, hydrolysis and deacetylation are then conducted to obtain the desired product 4'-thio UTP.

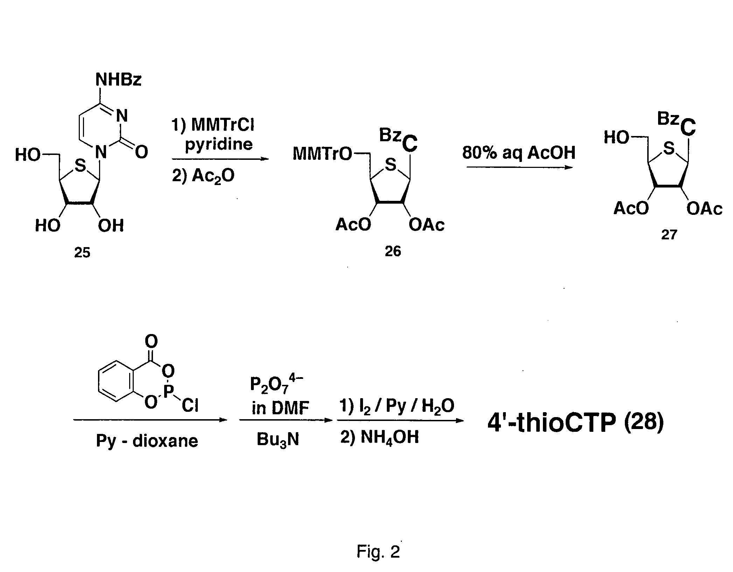

4'-Thio CTP 28 can be produced from N⁴-benzoyl-4'-thiocytidine 25 by the same process as the foregoing 4'-thio UTP (Fig. 2).

4'-Thio ITP can be produced from 4'-thiohypoxanthine by the same process as the foregoing 4'-thio UTP.

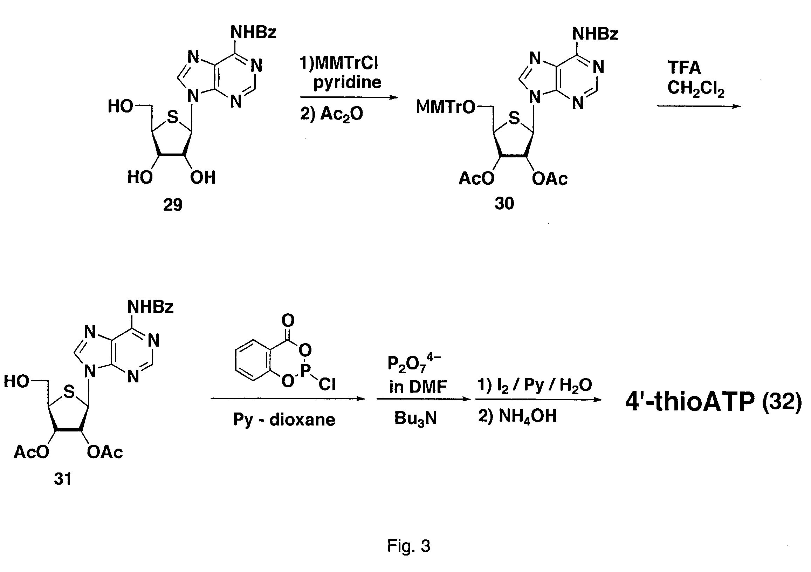

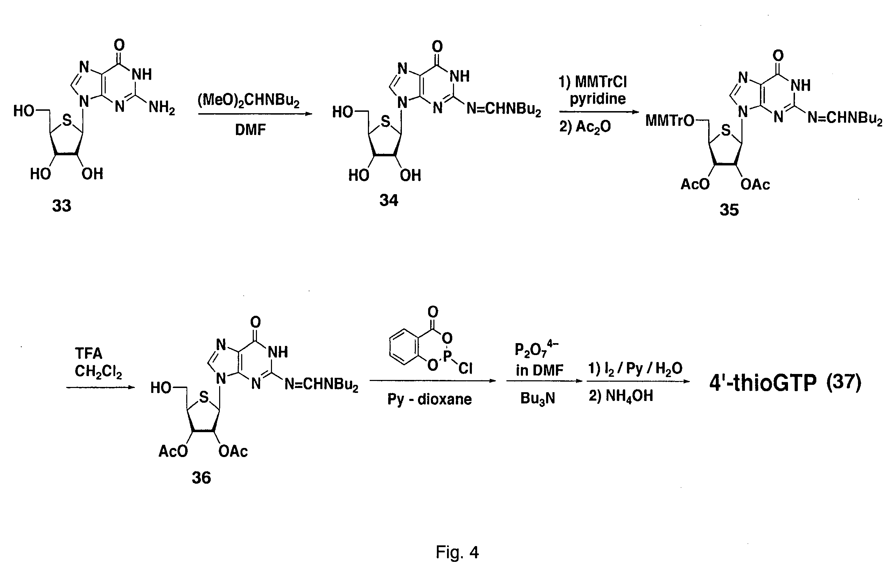

4'-Thio ATP and 4'-thio GTP can be produced by the schemes shown in Fig. 3 and Fig. 4. That is, after hydroxyl groups at the 2'- and 3'-positions and an amino group in the purine ring are appropriately protected, the compound is reacted with salicyl phosphorochloridite according to the method of Eckstein et al. (as mentioned above). The reaction product is then reacted with pyrophosphoric acid to obtain a cyclotriphosphite intermediate. Subsequently, iodo-oxidation, hydrolysis and deprotection are conducted to obtain 4'-thio ATP 32 and 4'-thio GTP 37.

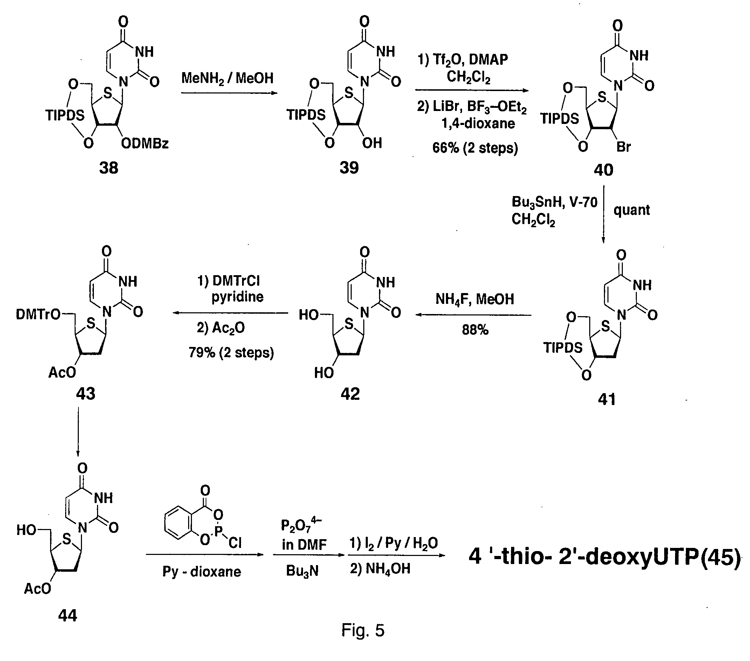

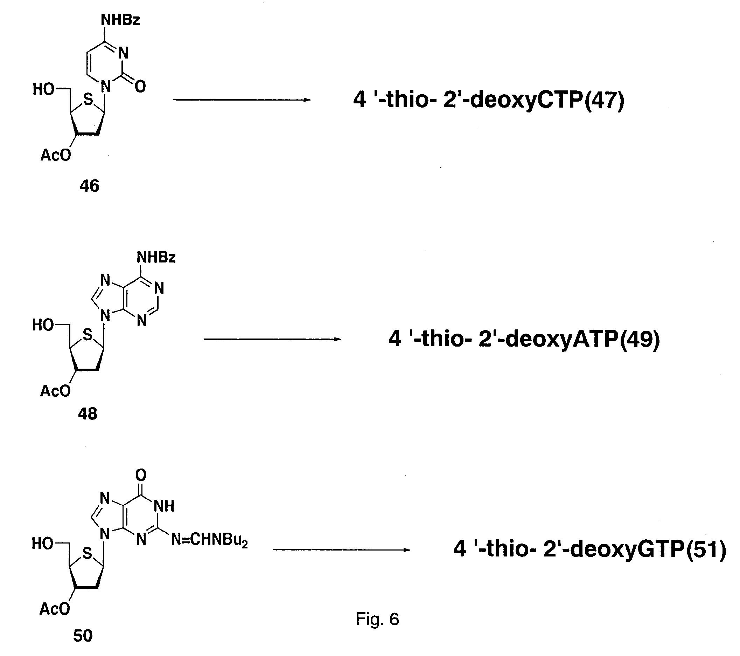

4'-Thio-2'-deoxyribonucleoside triphosphate can be produced in the same manner as 4'-thioribonucleoside triphosphate. A starting material, 4'-thio-2'-deoxyribonucleoside is reacted with salicyl phosphorochloridite. The resulting intermediate is reacted with pyrophosphoric acid, and iodo-oxidation, hydrolysis and deprotection are then conducted to obtain a desired product 4'-thio-2'-deoxyribonucleoside triphosphate (Figs. 5 and 6).

The 4'-thionucleotides of the invention can be used as a substrate for a reaction of synthesizing DNA or RNA using a polymerase. To confirm that the 4'-thioribonucleotide of the invention is recognized by RNA polymerase, the compound is contacted with RNA polymerase in the presence of an appropriate template, then determining whether the 4'-thioribonucleotide is incorporated into an oligomer. As demonstrated in Examples 15 and 16, it has been found that the 4'-thionucleotide synthesized according to the invention is recognized by T7 RNA polymerase and, like native nucleotides, incorporated into a synthetic oligomer chain.

In another aspect of the invention, the invention provides a process for producing an oligonucleotide using 4'-thionucleotides. The oligonucleotide can be produced by elongating an oligonucleotide chain with RNA or DNA synthetase, such as RNA polymerase or DNA polymerase, in the presence of the 4'-thionucleoside triphosphate of the invention. Various RNA polymerases derived from various organisms can be used as RNA synthetase. As DNA synthetase, DNA polymerase, reverse transcriptase, terminal deoxynucleotidyl transferase and the like derived from various organisms can be used. The conditions for the elongation reaction may vary with the polymerase used, and a skilled person can select appropriate reaction conditions. In the reaction, in addition to the 4'-thionucleoside triphosphate of the invention, native nucleoside triphosphates or modified nucleoside triphosphates known in the art may be present.

The oligonucleotide obtained by the process of the invention can be used as a diagnostic or therapeutic agent and an research reagent in the form of an antisense oligonucleotide, a ribozyme, a primer, an aptamer, an antigene, RNAi, siRNA, a probe or the like. Preferably, the oligonucleotide of the invention has a length of from about 6 to about 50 nucleotides. In a more preferred embodiment of the invention, the oligonucleotide has a length of from about 12 to about 20 nucleotides. The oligonucleotide may contain modified sugars, for example, sugar having a substituent at the 2'-position. It may also contain a nucleic acid other than adenine, guanine, cytosine, thymine and uracil, for example, hypoxanthine, 5-alkylcytidine, 5-alkyluridine, 5-halouridine, 6-azapyrimidine or 6-alkylpyrimidine. Further, it may contain an inter-nucleoside linkage other than a phosphodiester, for example, a phosphorothioate linkage.

The oligonucleotide of the invention is appropriately used in vitro and in vivo because it has high degree of nuclease resistance and heat stability. It is especially useful in gene therapy. Further, since RNA containing the 4'-thioribonucleoside of the invention is recognized by reverse transcriptase to synthesize cDNA, it is quite advantageous for use in in-vitro selection.

The entire contents of all patents and references cited in the present specification are hereby incorporated by reference. Further, the contents described in the specification and drawings of Japanese Patent Application No. 2002-242259, which is the basis for Convention priority of the present application, are hereby incorporated by reference.

### Examples

The invention is illustrated more specifically below by referring to Examples. However, these Examples do not limit the scope of the invention.

In Examples, the following abbreviations are used.
- Bz: benzoyl
- DMAP: 4-dimethylaminopyridine
- DMBz: dimethoxybenzoyl
- DMTr: 4,4'-dimethoxytrityl
- MMTr: 4-methoxytrityl
- Ms: methanesulfonyl
- Tf: trifluoroethanesulfonyl
- TFA: trifluoroacetic acid
- THF: tetrahydrofuran
- TIPDS: 1,1,3,3-tetraisopropyldicyloxane-1,3-diyl
- TMS: trimethylsilyl
- Ts: p-toluenesulfonyl

### Example 1

### Synthesis of 2,3,5-tri-O-p-methoxybenzyl-D-ribitol (3)

D-ribose (60 g, 0.4 mol) was dissolved in allyl alcohol (1.8 L, 26.4 mol), and conc. sulfuric acid (6.4 mL, 0.12 mol) was added at 0°C. Then, the solution was stirred overhead at room temperature. Subsequently, the reaction solution was neutralized by addition of sodium bicarbonate, and filtered with Celite. The filtrate was vacuum-dried by distilling off the solvent in vacuo to obtain a yellow oil residue. The residue dissolved in DMF (300 mL) was then cannulated in a THF (700 mL) solution of sodium hydride (64 g, 1. 6 mol) at 0°C over a period of 3 hours. The reaction solution was returned again to room temperature, and stirred for 4 hours. Thereafter, p-methoxybenzyl chloride (190 mL, 1.4 mol) was added dropwise at 0°C at a rate of 10 mL/15 min. When 100 mL was added dropwise, the solution was returned to room temperature, and the dropwise addition was carefully continued while observing a condition of H₂ occurrence. After completion of the dropwise addition, the solution was stirred at room temperature. After 13 hours, sodium hydride (4.0 g, 0.1 mol) and p-methoxybenzyl chloride (30 mL, 0.22 mol) were added, and the mixture was stirred for 24 hours. The reaction solution was neutralized with a saturated ammonium chloride aqueous solution. The solution was then diluted with ethyl acetate, and separated with water. The organic layer was washed with water (x3) and with a saturated sodium chloride aqueous solution (x1), and dried over Na₂SO₄. Then, after cotton plug filtration, the solvent was distilled off, and the resulting brown residue was vacuum-dried. The product was dissolved in chloroform (1.2 L), and oxygen was included. Water (800 mL) was added thereto, and palladium chloride (21.2 g, 0.12 mol) was added. The solution was stirred overhead at 50°C. After 9 hours, palladium chloride (7.0 g, 0.04 mol) was added. After 28 hours, the reaction solution was returned to room temperature, filtered with Celite, concentrated, then diluted with ethyl acetate, and separated with water. The organic layer was washed with water (x2) and with a saturated sodium chloride aqueous solution (x1), and dried over Na₂SO₄. Then, after cotton plug filtration, the solvent was distilled off, and the resulting brown residue was vacuum-dried. The residue was dried, and then dissolved in methanol (1.2 L). Sodium borohydride (30.3 g, 0.8 mol) was added in an argon atmosphere at 0°C, and the solution was stirred for 20 minutes. The solution was returned to room temperature, and stirred. After 1 hour, sodium borohydride (7.8 g, 0.2 mol) was added at 0°C. The solution was then returned to room temperature, and stirred. After 1.5 hours, the solvent of the reaction solution was distilled off, and the residue was azeotroped twice with methanol. The resulting residue was dissolved in ethyl acetate, and the solution was separated with water. The organic layer was washed with water (x2) and with a saturated sodium chloride aqueous solution (x1), and dried over Na₂SO₄. Then, after cotton plug filtration, the solvent was distilled off, and the residue was purified by silica gel chromatography (hexane:AcOEt = 5:1 -> 1:1) to obtain compound 3 (162.4 g, 79%) as colorless transparent oil.
¹H NMR (270 MHz, CDCl₃) δ: 7.30-6.81 (m, 12H, Ar), 4.66-4.40 (m, 6H, CH₂), 3.94-3.53 (m, 16H, H-1, 2, 3, 4, 5, MeOx3), 2.71 (br s, 1H, 4-OH), 2.36 (br s, 1H, 1-OH).

### Example 2

### Synthesis of 1,4-anhydro-2,3,5-tri-O-p-methoxybenzyl-4-thio-D-ribitol (5)

Mesyl chloride (122 mL, 1.6 mol) was added to a pyridine solution (900 mL) of compound 3 (162 g, 0.32 mol) in an argon atmosphere at 0°C, and the solution was stirred at the same temperature for 30 minutes. Ice was then added to the reaction solution, and the mixture was stirred for 20 minutes. The reaction solution was diluted with ethyl acetate, and separated with water. The organic layer was washed with a saturated sodium hydrogencarbonate aqueous solution (x2) and with a saturated sodium chloride aqueous solution (x1), and dried over Na₂SO₄. After cotton plug filtration, the solvent was distilled off in vacuo, and the residue was azeotroped three times with toluene. The residue was vacuum-dried, and then dissolved in MEK (1L) in an argon atmosphere. Lithium bromide (278 g, 3.2 mol) was added at room temperature, and the mixture was heat-refluxed. After 12 hours, the reaction solution was returned to room temperature, diluted with ethyl acetate, and separated with water. The organic layer was washed with water (x2) and with a saturated sodium chloride aqueous solution (x1), and dried over Na₂SO₄. After cotton plug filtration, the solvent was distilled off in vacuo, and the resulting residue was vacuum-dried, and then dissolved in DMF (1 L) in an argon atmosphere. Sodium sulfide 9-hydrate (92.2 g, 0.38 mol) was added at room temperature, and the mixture was stirred at 100°C for 30 minutes. Subsequently, the reaction solution was returned to room temperature, diluted with ethyl acetate, and separated with water. The organic layer was washed with water (x2) and with a saturated sodium chloride aqueous solution (x1), and dried over Na₂SO₄. After cotton plug filtration, the solvent was distilled off in vacuo, and the resulting residue was coarsely purified by silica gel chromatography (hexane:AcOEt = 10:1 -> 1:1), and then recrystallized from hexane and ethyl acetate. Compound 5 (69.1 g, 42%) was obtained as a white crystal.
¹H NMR (270 MHz, CDCl₃) δ: 7.26-6.81 (m, 12H, Ar), 4.52-4.38 (m, 6H, CH₂), 4.01-3.95 (m, 1H, H-2), 3.91 (t, 1H, H-3, J_{3, 2} =4.0, J_{3, 4} =4.0 Hz), 3.80 (s, 9H, MeOx3), 3.66-3.60 (m, 1H, H-4), 3.45-3.40 (m, 2H, H-5a, H-5b), 3.02 (dd, 1H, H-1a, J_{1a,1b} = 10.6, J_{1a, 2} =6.5 Hz), 2.87 (dd, 1H, H-1b, J_{1b, 1a} =10.6, J_{1b, 2} =5.2 Hz).

### Example 3

### Synthesis of 1,4-anhydro-2-O-(4-methoxybenzoyl)-3,5-O-(1,1,3,3-tetraisopropyldisiloxane-1,3-diyl)-4-thio-D-ribitol (13b)

4-Methoxybenzoyl chloride (723 µL, 5.1 mmol) was added to a pyridine solution (15 mL) of compound 12 (997 mg, 2.5 mmol) in an argon atmosphere at 0°C, and the mixture was stirred at room temperature for 18.5 hours. Ice was added to the reaction solution, and the mixture was stirred for 10 minutes. Then, the solution was diluted with ethyl acetate, and separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo, and the residue was azeotroped three times with toluene. The resulting residue was purified by silica gel chromatography (hexane:AcOEt = 50:1 -> 35:1) to obtain compound 13b (1.3 g, 96%) as colorless oil.
FAB-LRMS m/z 527 (MH⁺).
FAB-HLRS Calcd for C₂₅H₄₂O₆SSi₂ (MH⁺): 527.2319. Found: 527.2311.
¹H NMR (400 MHz, CDCl₃) δ: 7.95-6.61 (m, 4H, Ar), 5.71-5.69 (m, 1H, H-2), 4.35 (dd, 1H, H-3 J_{3, 2} =3.5, J_{3, 4} =9.4 Hz), 4.12 (dd, 1H, H-5a, J_{5a, 4} =2.9, J_{5a, 5b} =12.6 Hz), 3.98 (dd, 1H, H-5b, J_{5b, 4} =3.2, J_{5b, 5a} =12.6 Hz), 3. 69 (m, 1H, H-4), 3. 24 (dd, 1H, H-1β, J_{1β, 2}= 3.2, J_{1β, 1α} =12.6 Hz), 3.04 (m, 6H, Me₂N), 2.91 (d, 1H, H-1α, J_{1α, 1β} =12.6 Hz), 1.13-0.87 (m, 28H, TIPDS).
¹³C NMR (100 MHz, CDCl₃) δ: 166.40, 153.59, 131.73, 117.34, 110.94, 110.81, 75.95, 75.21, 60.16, 51.29, 40.44, 31.60, 17.84, 17.77, 17.70, 17.66, 17.52, 17.45, 13.87, 13.75, 13.15, 13.09.

### Example 4

### Synthesis of 1,4-anhydro-2-O-(4-dimethylaminobenzoyl)-3,5-O-(1,1,3,3-tetraisopropyldisiloxane-1,3-diyl)-4-thio-D-ribitol (13c)

Thionyl chloride (933 µL, 12.8 mmol) was added to a methylene chloride solution (40 mL) of 4-dimethylaminobenzoic acid (1.1 g, 6. 4 mmol) in an argon atmosphere, and the mixture was heated to reflux with stirring for 2 hours. The temperature was returned to room temperature, and the solvent was distilled off in vacuo. The resulting residue (533 mg) was added to a pyridine solution (5 mL) of compound 12 (375 mg, 0.96 mmol) at 0°C in an argon atmosphere, and the mixture was stirred at room temperature for 8 hours. After 8 hours, the foregoing residue (355 mg) was added again. After 21 hours, pyridine (5 mL) was added, and the mixture was heated at 50°C for 6 hours. Ice was added to the reaction solution, and the mixture was stirred for 10 minutes. The reaction solution was then diluted with ethyl acetate, and separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo, and azeotroped three times with toluene. The resulting residue was purified by silica gel chromatography (hexane:AcOEt = 50:1 -> 20:1) to obtain compound 13c (476 mg, 92%) as colorless oil.
FAB-LRMS m/z 540(MH⁺).
FAB-HLRS Calcd for C₂₆H₄₅NO₅SSi₂ (MH⁺): 540.2635. Found: 540.2637.
¹H NMR (400 MHz, CDCl₃) δ: 7.95-6.61 (m, 4H, Ar), 5.71-5.69 (m, 1H, H-2), 4.35 (dd, 1H, H-3 J_{3, 2} =3.5, J_{3, 4} =9.4 Hz), 4.12 (dd, 1H, H-5a, J_{5a, 4} =2.9, J_{5a, 5b} =12.6 Hz), 3.98 (dd, 1H, H-5b, J_{5b, 4} =3.5, J_{5b, 5a} =12.6 Hz), 3.69 (m, 1H, H-4), 3.24 (dd, 1H, H-1β, J_{1β, 2}= 3.2, J_{1β, 1α} =12.6 Hz), 3.04 (m, 6H, Me₂N) 2.91 (d, 1H, H-1α, J_{1α, 1β} =12.6 Hz), 1.13-0.87 (m, 28H, TIPDS).
¹³C NMR (100 MHz, CDCl₃) δ: 166.40, 153.59, 131.73, 117.34, 110.94, 110.81, 75.95, 75.21, 60.16, 50.03, 40.44, 31.60, 17.84, 17.77, 17.71, 17.67, 17.52, 17.45, 13.87, 13.75, 13.15, 13.09.

### Example 5

### Synthesis of 1,4-anhydro-2-O-(4-methoxybenzoyl)-3,5-O-(1,1,3,3-tetraisopropyldisiloxane-1,3-diyl)-4-thio-D-ribitol-1-oxide (14b)

Ozone gas was bubbled in a methylene chloride solution (20 mL) of compound 13b (1.1 g, 2.3 mmol) at -78°C for 20 minutes. Argon was bubbled in the reaction solution until the ozone odor disappeared, and the temperature was then raised to room temperature. After the solvent was distilled off in vacuo, the residue was purified by silica gel chromatography (hexane:AcOEt = 4:1 -> 1:3) to obtain compound 14b (1.0 g, 82%) as a colorless sticky product.
FAB-LRMS m/z 543 (MH⁺).
FAB-HLRS Calcd for C₂₅H₄₂O₇SSi₂ (MH⁺): 543.2253. Found: 543.2251.
¹H NMR (400 MHz, CDCl₃) δ: 8.06-6.90 (m, 4H, Ar), 5.84-5.82 (m, 1H, H-2), 4.61 (d, 1H, H-5a, J_{5a, 5b} =12.9 Hz), 4.23 (dd, 1H, H-5b, J_{5b, 4} =2.9, J_{5b, 5a} =12.9 Hz), 4.17 (dd, 1H, H-3 J_{3, 2} =3.5, J_{3, 4} =12.0 Hz), 3.86 (s, 3H, MeO), 3.61(dd, 1H, H-1β, J_{1β, 2}= 5.3, J_{1β, 1α} =15.5 Hz), 3.48 (dd, 1H, H-4, J_{4, 5b} =2.1, J_{4, 3} =12.0 Hz), 2.93 (d, 1H, H-1α, J_{1α, 1β} =15.5 Hz), 1.09-0.87 (m, 28H, TIPDS).
¹³C NMR (100 MHz, CDCl₃) δ: 165.42, 163.84, 132.20, 122.24, 113.96, 73.31, 73.15, 68.48, 55.79, 55.75, 54.59, 17.72, 17.64, 17.58, 17.54, 17.53, 17.49, 17.31, 17.30, 13.81, 13.51, 13.02, 13.00.

### Example 6

### 1-[2-O-(4-methoxytrityl)-3,5-O-(1,1,3,3-tetraisopropyldisiloxane-1,3-diyl)-4-thio-β-D-ribofuranosyl]uracil (15b)

Triethylamine (1.0 mL , 7.3 mmol) and TMSOTf (2.6 mL, 14.6 mmol) were added to a toluene suspension (20 mL) of uracil (408 mg) in an argon atmosphere at room temperature, and the reaction solution was stirred until a two-layer solution was formed. Methylene chloride (10 mL) was added to this reaction solution to form a monolayer solution. This reaction solution was added dropwise to a methylene chloride solution (20 mL) of compound 14b (987 mg, 1.8 mmol) at room temperature over a period of 15 minutes. Subsequently, a toluene solution (10 mL) of triethylamine (1.0 mL, 7.3 mmol) was added dropwise at room temperature over a period of 5 minutes. Water was added to the reaction solution, and the mixture was stirred for 10 minutes. The solution was then diluted with ethyl acetate, and separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo. The residue was purified by silica gel chromatography (hexane:AcOEt = 49:1 -> 1:1) to obtain compound 15b (904 mg, 77%) as a white foam.
FAB-LRMS m/z 637 (MH⁺).
FAB-HLRS Calcd for C₂₉H₄₄N₂O₈SSi₂ (MH⁺): 637.2435. Found: 637.2435.
¹H NMR (400 MHz, CDCl₃) δ: 9.28 (br s, 1H, NH), 8.22 (dd, 1H,H-6, J_{6, 5} =8.2), 8.02-6.94 (m, 4H, Ar), 6.01 (s, 1H, H-1'), 5.76 (dd, 1H, H-5, J_{5, 6} =8.2, J_{5, NH} =2.1 Hz), 5.62 (d, 1H, H-2', J_{2', 3'} =3.5), 4.45 (dd, 1H, H-3' J_{3', 2'} =3.5, J_{3', 4'} =9.4 Hz), 4.18-4.06 (m, 2H, H-5'a, H-5'b), 3.87 (s, 3H, MeO), 3.73-3.71 (m, 1H, H-4'), 1.15-0.86 (m, 28H, TIPDS).
¹³C NMR (100 MHz, CDCl₃) δ: 164.01, 163.38, 163.10, 150.13, 140.653, 131.79, 121.73, 113.53, 102.19, 102.17, 78.06, 71.24, 62.54, 57.92, 55.40, 55.39, 50.69, 17.47, 17.36, 17.34, 17.27, 16.99, 16.96, 16.83, 16.79, 13.34, 13.18, 13.10, 13.48.

### Example 7

### 1-[5-O-(4-methoxytrityl)-2,3-O-diacetyl-4-thio-β-D-ribofuranosyl]uracil (20)

In an argon atmosphere, 4-methoxytrityl chloride (232 mg, 0.75 mmol) was added to a pyridine (4 mL) solution of 1-(4-thio-β-D-ribofuranosyl)uracil (131 mg, 0.5 mmol; produced by deprotecting compound 15 with NH₄F/MeOH and MeNH₂/MeOH), and the mixture was stirred at room temperature for 14 hours. Acetic anhydride (188 µL, 2 mmol) and DMAP (5 mg, 0.05 mmol) were added in an argon atmosphere, and the mixture was stirred at room temperature for 2 hours. Methanol was added, and the solution was stirred for 30 minutes. The solvent was distilled off in vacuo from the reaction solution. The residue was dissolved in ethyl acetate, and the solution was separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo. The residue was purified by silica gel chromatography (hexane:AcOEt = 2:1 -> 1:1) to obtain compound 20 (214 mg, 70%) as a colorless transparent solid.
¹H NMR (270 MHz, CDCl₃) δ: 8.07 (br s, 1H, NH), 7.77 (d, 1H,H-6, J_{6, 5} =7.9), 7.47-6.85 (m, 14H, MMTr), 6.37 (d, 1H, H-1', J_{1',2'} =7.3), 5.68 (dd, 1H, H-2', J_{2',1'}=7.3, J_{2',3'} =4.0), 5.54-5.51 (m, 1H, H-3'), 5.62 (dd, 1H, H-5, J_{5, 6} =7.9, J_{5, NH}=2.6Hz), 3.81 (s, 3H, MeO), 3.62-3.53 (m, 2H, H-5'a, H-4'), 3.40-3.35 (m, 1H, H-5'b), 2.12-2.00 (m, 6H, AcOx2).

### Example 8

### 1-(2,3-O-diacetyl-4-thio-β-D-ribofuranosyl)uracil (21)

Compound 20 (199 mg, 0.32 mmol) was dissolved in a 80% acetic acid aqueous solution, and the mixture was stirred at room temperature for 11 hours. The reaction solution was added dropwise to a saturated sodium hydrogencarbonate aqueous solution, and the mixture was separated with ethyl acetate. The aqueous layer was extracted seven times with chloroform. The organic layer was washed with a saturated sodium chloride aqueous solution (x1), and dried over Na₂SO₄. After cotton plug filtration, the solvent was distilled off in vacuo. The residue was purified by silica gel chromatography (hexane:AcOEt = 2:1 -> AcOEt) to obtain compound 21 (93 mg, 84%) as a white foam.
¹H NMR (270 MHz, CDCl₃) δ: 8.23 (br s, 1H, NH), 8.08 (d, 1H,H-6, J_{6, 5} =7.9), 6.36 (d, 1H, H-1', J_{1', 2'} =7.3), 5.85 (m, 1H, H-5), 5.69 (dd, 1H, H-2', J_{2', 1'} =7.3, J_{2', 3'} =4.0), 5.49 (m, 1H, H-3'), 4.16-4.04 (m, 1H, H-5'a), 3.86-3.82 (m, 1H, H-5'b), 3.56-3.55 (m, 1H, H-4'), 2.42 (br s, 1H, OH), 2.17 (s, 3H, AcO), 2.06 (s, 3H, AcO)

### Example 9

### 4'-Thiouridine 5'-triphosphate (24)

Compound 21 (89 mg, 0.26 mmol) was azeotroped with pyridine, and dissolved in pyridine (260 µL). Dioxane (780 µL) was added in an argon atmosphere, and the solution was stirred at room temperature. A dioxane solution of salicyl phosphorochloridite (58 mg, 2.9 mmol) was added, and the mixture was stirred for 10 minutes. A DMF solution (780 µL) of 0.5 M bisbutylammonium pyrophosphate was added, and butylamine (260 µL) was quickly added, followed by stirring for 10 minutes. 1% Iodine (5 mL) was added in pyridine/water (98/2, v/v), and the mixture was stirred for 5 minutes. Several drops of 5% sodium hydrogensulfite aqueous solution were added, and the mixture was stirred for 40 minutes. Aqueous ammonia (8 mL) was added to the reaction solution, and the mixture was stirred for 3.5 hours. The solvent was distilled off in vacuo. The residue was dissolved in 300 mL of water, and the solution was purified by ion exchange chromatography (H₂O -> 1N TEAB), and then purified via a salt exchange column (H⁺ type). The solvent was distilled off in vacuo. The residue was dissolved in 5 mL of water, and the solution was purified via a salt exchange column (Na⁺ type). The solvent was distilled off in vacuo. Compound 24 (80 mg, 55%) was obtained as pale yellow oil.
FAB-HRMS Calcd for C₉H₁₂N₂Na₃O₁₄P₃S (M⁺): 563.8893. Found: 563.8892
³¹P-NMR(108 MHz, D₂O) δ: -5.68 (d, J = 20 Hz), -10.56 (d, J = 20 Hz), -21.27 (t, J = 20 Hz).

### Example 10

### Synthesis of 4'-thio CTP (28) (Fig. 2)

### N⁴-benzoyl-1-[5-O-(4-methoxytrityl)-2,3-O-diacetyl-4-thio-β-D-ribofuranosyl]cytosine (26)

4-Methoxytrityl chloride (383 mg, 1.2 mmol) was added to a pyridine (10 mL) solution of compound 25 (302 mg, 0.83 mmol) in an argon atmosphere, and the mixture was stirred at room temperature for 9 hours. Acetic anhydride (313 µL, 3.32 mmol) was added in an argon atmosphere, and the mixture was stirred at room temperature for 3 hours. Methanol was added, and the solution was stirred for 5 minutes. The solvent was distilled off in vacuo from the reaction solution. The residue was dissolved in ethyl acetate, and the solution was separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo. The residue was purified by silica gel chromatography (hexane:AcOEt = 2:1 -> AcOEt) to obtain compound 26 (579 mg, 97%) as a white foam.
FAB-LRMS m/z 720 (MH⁺)
¹H NMR (400 MHz, CDCl₃) δ: 8.86 (br s, 1H, NH), 8.32 (d, 1H,H-6, J_{6, 5} =7.6), 7.89-7.88 (m, 2H, o-Bz), 7.61-7.27 (m, 15H, MMTr, m-Bz, p-Bz, H-5), 6.90-6.87 (m, 2H, MMTr), 6.53 (d, 1H, H-1', J_{1', 2'} =6.2), 5.70-5.68 (m, 1H, H-3'),5.17 (m, 1H, H-2'), 3.82 (s, 3H, MeO), 3.63-3.56 (m, 2H, H-5'a, H-4'), 3.46-3.44 (m, 1H, H-5'b),2.08 (s, 3H, AcO), 2.06 (s, 3H, AcO).

### N⁴-benzoyl-1-[2,3-O-diacetyl-4-thio-β-D-ribofuranosyl]cytosine (27)

Compound 26 (420 mg, 0.58 mmol) was dissolved in 80% acetic acid aqueous solution, and the mixture was stirred at room temperature for 24 hours. The reaction solution was added dropwise to a saturated sodium hydrogencarbonate aqueous solution, and the resulting solution was separated with chloroform. The aqueous layer was extracted three times with chloroform. The organic layer was washed with a saturated sodium hydrogencarbonate aqueous solution (x1) and with a saturated sodium chloride aqueous solution (x1), and dried over Na₂SO₄. After cotton plug filtration, the solvent was distilled off in vacuo. The residue was purified by silica gel chromatography (hexane:AcOEt = 1:1 -> AcOEt -> acetone:AcOEt = 1:3) to obtain compound 27 (239 mg, 92%) as a white foam.
FAB-LRMS m/z 448 (MH⁺)
¹H NMR (400 MHz, CDCl₃) δ: 9.11 (br s, 1H, NH), 8.75 (d, 1H,H-6, J_{6, 5} =7.6), 7.86-7.84 (m, 2H, o-Bz), 7.60-7.43 (m, 4H, H-5, m-Bz, p-Bz), 6.45 (d, 1H, H-1', J_{1', 2'} =6.7), 5.83-5.80 (m, 1H, H-3'), 5.58-5.68 (m, 1H, H-2'), 4.45 (br s, 1H, OH), 4.07-4.04 (m, 1H, H-5'a), 3.90-3.86 (m, 1H, H-5'b), 3.61-3.60 (m, 1H, H-4'), 2.11 (s, 3H, AcO), 2.01 (s, 3H, AcO)

### 4'-Thiocytidine 5'-triphosphate (28)

Compound 27 (71 mg, 0.16 mmol) was treated with salicyl phosphorochloridite (50 mg, 0.24 mmol) as in the synthesis of compound 24 to obtain compound 28 (68 mg, 75%) as a white powder. FAB-HRMS Calcd for C₉H₁₂N₃Na₃O₁₃P₃S (M-H)⁺: 563.8893. Found: 563.8892.
³¹P-NMR(108 MHz, D₂O) δ: -6.28 (d, J = 20 Hz), -10.49 (d, J = 20 Hz), -21.24 (t, J = 20 Hz).

### Example 11

### Synthesis of 4'-thio ATP (32) (Fig. 3)

### N⁶-benzoyl-9-[5-O-(4-methoxytrityl)-2,3,0-diacetyl-4-thio-β-D-ribofuranosyl]adenine (30)

4-Methoxytrityl chloride (92 mg, 0.3 mmol) was added to a pyridine (2 mL) solution of compound 29 (77 mg, 0.2 mmol) in an argon atmosphere, and the mixture was stirred at room temperature. After 12 hours, further 4-methoxytrityl chloride (92 mg, 0.3 mmol) was added, and the solution was stirred at room temperature. After 24 hours, acetic anhydride (94 µL, 1.0 mmol) was added to the reaction solution, and the mixture was stirred at room temperature for 12 hours. Ice water was added to the reaction solution, and the resulting mixture was stirred for 5 minutes. The solvent was then distilled off in vacuo. The residue was dissolved in ethyl acetate, and the solution was separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo. The residue was purified by silica gel chromatography (hexane:AcOEt = 1:2 -> AcOEt) to obtain compound 30 (100 mg, 68%) as a colorless glassy product.
FAB-LRMS m/z 730 (MH⁺).
¹H NMR (270 MHz, CDCl₃) δ: 8.99 (br s, 1H), 8.74 (s, 1H), 8.24 (s, 1H), 8.02 (m, 2H), 7.64-7.28 (m, 15H), 6.86 (m, 2H), 6.31 (d, 1H, J = 7.3), 6.02 (m, 1H), 5.72 (m, 1H), 3.80 (s, 3H), 3.68 (m, 1H), 3.55 (m, 2H), 2.14 and 1.98 (each s, each 3H).

### N⁶-benzoyl-9-(2,3-O-diacetyl-4-thio-β-D-ribofuranosyl)adenine (31)

Trifluoroacetic acid (20 µL) was added to a methylene chloride (2 mL) solution of compound 30 (104 mg, 0.14 mmol) in an argon atmosphere at 0° C. The temperature was then elevated to room temperature, and the mixture was stirred for 5 hours. Triethylamine (140 µL) was added to the reaction solution, and the solvent was distilled off in vacuo. The residue was purified by silica gel chromatography (AcOEt:acetone = 0:1 -> 1:2) to obtain compound 31 (53 mg, 79%) as a white foam.
FAB-LRMS m/z 472 (MH⁺).
¹H NMR (270 MHz, CDCl₃) δ: 8.98 (br s, 1H), 8.81 (s, 1H), 8.19 (s, 1H), 8.00 (m, 2H), 7.59-7.48 (m, 3H), 6.28 (m, 1H), 6.14 (d, 1H, J = 7.3), 5.69 (m, 1H), 4.75 (m, 1H), 4.09 (m, 1H), 3.93 (m, 1H), 3.66 (m, 1H), 2.16 and 1.95 (each s, each 3H).

### 4'-Thioadenosine 5'-triphosphate (32)

Compound 31 (53 mg, 0.11 mmol) was treated with salicyl phosphorochloridite (25 mg, 0.12 mmol) as in the synthesis of compound 24 to obtain compound 32 (45 mg, 68%) as a white powder.
FAB-HRMS Calcd for C₁₀H₁₃N₅Na₃O₁₂ P₃S (MH⁺): 589.9265. Found: 589.9268.
³¹P-NMR(108 MHz, D₂O) δ: -5.30 (d, J = 20 Hz), -10.39 (d, J = 20 Hz), -20.88 (t, J =20 Hz).

### Example 12

### Synthesis of 4'-thio GTP (37) (Fig. 4)

### N²-(N,N'-dibutylaminomethylene)-9-(4-thio-β-D-ribofuranosyl)guanine (34)

N,N'-dibutylformamidedimethylacetal (302 µL, 0.92 mmol) was added to a DMF (3 mL) solution of compound 33 (106 mg, 0.46 mmol) in an argon atmosphere, and the mixture was stirred at room temperature for 3.5 hours. After the solvent was distilled off in vacuo from the reaction solution, the residue was purified by silica gel chromatography (20% MeOH in CHCl₃) to obtain compound 34 (139 mg, 69%) as a white solid.
FAB-HRMS Calcd for C₁₉H₃₀N₆O₄S (MH⁺): 439.2153. Found: 439. 2156.
¹H NMR (400 MHz, DMSO-d₆) δ: 8.54 (s, 1H, N=CH), 8.08 (s, 1H, H-8), 5.74(d, 1H, H-1', J_{1', 2'} =6.6), 5.48(d, 1H, 2'-OH, J_{2'-OH',} _{2'} =6.0), 5.31(d, 1H, 3'-OH, J_{3'-OH', 3'} =4.5), 5.14(m, 1H, 5'-OH), 4.60-4.55 (m, 1H, H-2'), 4.19-4.17 (m, 1H, H-3'), 3.80-3.55 (m, 2H, H-5'a, b), 3.48-3.48 (m, 5H, H-4', n-Bu), 1.56-0.82(m, 14H, n-Bu).

### N²-(N,N'-dibutylaminomethylene)-9-[5-O-(4-methoxytrityl)-2,3-O-diacetyl-4-thio-β-D-ribofuranosyl]guanine (35)

4-Methoxytrityl chloride (143 mg, 0.47 mmol) was added to a pyridine (4 mL) solution of compound 34 (135 mg, 0.31 mmol) in an argon atmosphere, and the mixture was stirred at room temperature. After 23 hours, 4-methoxytrityl chloride (47 mg, 0.16 mmol) was further added, and the solution was stirred at room temperature. After 71 hours, acetic anhydride (117 µL, 1.24 mmol) was added to the reaction solution, and the mixture was stirred at room temperature for 3 hours. Methanol was added to the reaction solution, and the mixture was stirred for 5 minutes. Then, the solvent was distilled off in vacuo. The residue was dissolved in ethyl acetate, and the solution was separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo. The residue was purified by silica gel chromatography (hexane:AcOEt = 1:2 -> AcOEt) to obtain compound 35 (158 mg, 64%) as a white foam.
FAB-HRMS Calcd for C₄₃H₅₀N₆O₇S (MH⁺): 795. 3528. Found: 795. 3519.
¹H NMR (400 MHz, CDCl₃) δ: 8.64 (s, 1H, N=CH), 8.44(br s, 1H,NH), 8.00(s, 1H,H-8), 7.47-7.44 (m, 2H, o-Bz), 7.35-7.21 (m, 14H, MMTr, m-Bz, p-Bz), 6.86-6.83 (m, 2H, MMTr), 6.08-6.05 (m, 1H , H-3'), 5.87(d, 1H, H-1', J_{1', 2'} =4.3), 5.56-5.12 (m, 1H, H-2'), 3.80 (s, 3H, MeO), 3.77-3.70 (m, 1H, H-5'a), 3.48-3.29 (m, 6H, H-4', n-Bu, H-5'b), 2.08 (s, 3H, AcO), 2.01 (s, 3H, AcO), 1.65-0.94(m, 14H, n-Bu).

### N²-(N,N'-dibutylaminomethylene)-9-(2,3,O-diacetyl-4-thio-β-D-ribofuranosyl)guanine (36)

Trifluoroacetic acid (30 µL) was added to a methylene chloride (3 mL) solution of compound 35 (150 mg, 0.19 mmol) at 0°C in an argon atmosphere. The temperature was then elevated to room temperature, and the mixture was stirred for 5 hours. Subsequently, triethylamine (120 µL) was added to the reaction solution, and the solvent was distilled off in vacuo. The residue was purified by silica gel chromatography (AcOEt: acetone = 3:1 -> 1:1) to obtain compound 36 (93 mg, 92%) as a white foam.
FAB-HRMS Calcd for C₂₃H₃₄N₆O₆S (MH⁺): 523.2339. Found: 523. 2346.
¹HNMR (400 MHz, DMSO-d₆) δ:8.60 (s, 1H, N=CH), 8.07 (s, 1H,H-8), 6.13-6.11 (m, 1H , H-2'), 6.06 (d, 1H, H-1', J_{1', 2'} =6.2), 5.69-5.67 (m, 1H, H-3'), 5.43-5.41 (m, 1H, 5'-OH), 3.84-3.79 (m, 1H, H-5'a), 3.74-3.66 (m, 1H, H-5'b), 3.59-3.54 (m, 1H, H-4'), 3.48-3.34 (m, 4H, n-Bu), 2.08 (s, 3H, AcO), 1.97 (s, 3H, AcO), 1.61-0.89 (m, 14H, n-Bu).

### 4'-Thioguanosine 5'-triphosphate (37)

Compound 36 (52 mg, 0.1 mmol) was treated with salicyl phosphorochloridite (20 mg, 0.15 mmol) as in the synthesis of compound 24 to obtain compound 37 (25 mg, 41%) as a white powder.
FAB-HRMS Calcd for C₁₀H₁₄N₅Na₃O₁₃ P₃S (MH⁺): 605.9215. Found: 605.9214.
³¹P-NMR(108 MHz, D₂O) δ: -5.15 (d, J = 20 Hz), -10.41 (d, J = 20 Hz), -20.79 (t, J =20 Hz).

### Example 13

### Synthesis of 4'-Thio-2'-deoxy UTP (45) (Fig. 5)

### 1-[2-Bromo-2-deoxy-3,5-O-(1,1,3,3-tetraisopropyldisiloxane-1,3-diyl)-4-thio-β-D-ribofuranosyl]uracil (40)

Dimethylaminopyridine (733 mg, 6.0 mmol) and trifluoromethanesulfonic anhydride (0.5 mL, 3.0 mmol) were added to a dichloromethane (15 mL) solution of compound 39 (757 mg, 1.5 mmol), and the mixture was stirred at room temperature for 10 minutes. Ethyl acetate was added to the reaction solution, and the mixture was separated with water. The organic layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo. The resulting residue was azeotroped with toluene, and then dissolved in 1,4-dioxane (15 mL). Lithium bromide (195 mg, 2.25 mmol) and a boron trifluoride-diethyl ether complex (285 µL, 2.25 mmol) were added to the solution, and the mixture was heated at 50°C for 1.5 hours. Ethyl acetate was added to the reaction solution, and the mixture was separated with water. The organic layer was washed with a saturated sodium chloride aqueous solution (x1), and dried over Na₂SO₄. After cotton plug filtration, the solvent was distilled off in vacuo. The residue was purified by silica gel chromatography (hexane:AcOEt = 2:1 -> 1:1) to obtain compound 40 (561 mg, 66%) as a white foam.
FAB-LRMS m/z 566, 568 (MH⁺)
¹H NMR (270 MHz, CDCl₃) δ: 8.65 (br s, 1H), 8.41 (d, 1H, J =8.6), 5.99 (s, 1H), 5.68 (dd, 1H, J = 2.0 and 8.6), 4.31(m, 1H), 4.14-3.98 (m, 3H), 3.67 (m, 1H), 1.57-0.92 (m, 28H).

### 1-[2-Deoxy-3,5-O-(1,1,3,3-tetraisopropyldisiloxane-1,3-diyl)-4-thio-β-D-ribofuranosyl]uracil (41)

Tributyltin hydride (371 µL, 1.38 mmol) and then V-70 (57 mg, 0.18 mmol) were added to a dichloromethane (15 mL) solution of compound 40 (519 mg, 0.92 mmol), and the mixture was stirred at room temperature for 10 minutes. The solvent was distilled off, and the residue was purified by silica gel chromatography (hexane:AcOEt = 10:1 -> 2:1) to obtain compound 41 (440 mg, 98%) as a white foam.
FAB-LRMS m/z 487 (MH⁺)
¹H NMR (270 MHz, CDCl₃) δ: 8.62 (br s, 1H), 8.28 (d, 1H, J =8.6), 5.97 (d, 1H, J = 6.6), 5.68 (dd, 1H, J = 2.0 and 8.6), 4.37(m, 1H), 4.11 (dd, 1H, J = 3.3 and 12.5), 3.30 (m, 1H), 2.45 (m, 1H), 2.24 (m, 1H), 1.61-0.94 (m, 28H).

### 1-(2-Deoxy-4-thio-β-D-ribofuranosyl)uracil (42)

Ammonium fluoride (666 mg, 18 mmol) was added to a methanol (20 mL) solution of compound 41 (437 mg, 0.9 mmol), and the mixture was heat-refluxed at 70°C. After 1 hour, the solvent was distilled off, and the residue was purified by silica gel chromatography (MeOH in CHCl₃ = 5% -> 25%) to obtain compound 42 (193 mg, 88%) as a white solid.
FAB-LRMS m/z 245 (MH⁺)
¹H NMR (270 MHz, DMSO-d₆ + D₂O) δ: 7.95 (d, 1H, J = 7.9), 6.22 (dd, 1H, J = 7.4 and 8.0), 5.65 (d, 1H, J = 7.9), 4.31 (m, 1H), 3.54 (m, 1H), 3.26 (m, 1H), 2.13 (m, 2H).

### 1-[2-Deoxy-5-O-(4,4'-dimethoxytrityl)-3-O-acetyl-4-thio-β-D-ribofuranosyl)uracil (43)

4,4'-Dimethoxytrityl chloride (203 mg, 1.5 mmol) was added to a pyridine (3 mL) solution of compound 42 (122 mg, 0.5 mmol) in an argon atmosphere, and the mixture was stirred at room temperature for 12 hours. Acetic anhydride (141 µL, 3.32 mmol) was added in an argon atmosphere, and the mixture was stirred at room temperature for 1 hour. Methanol was added, and the mixture was stirred for 5 minutes. The solvent was distilled off in vacuo from the reaction solution. The residue was dissolved in ethyl acetate, and the solution was separated with water. The aqueous layer was washed with a saturated sodium hydrogencarbonate aqueous solution (x3) and with a saturated sodium chloride aqueous solution (x1), and dried over Na₂SO₄. After cotton plug filtration, the solvent was distilled off in vacuo. The residue was purified by silica gel chromatography (hexane:AcOEt = 2:1 -> 1:2) to obtain compound 43 (233 mg, 79%) as a white foam.
FAB-LRMS m/z 589 (MH⁺)
¹H NMR (270 MHz, CDCl₃) δ: 8.24 (br s, 1H), 7.65 (d, 1H, J = 8.0), 7.42-7.25 (m, 9H), 6.83 (m, 4H), 6.44 (m, 1H), 5.41 (m, 2H), 3.78 (s, 6H), 3.59 (m, 1H), 3.48 (m, 1H), 3.29 (m, 1H), 2.46 (m, 1H), 2.11 (m, 1H), 2.09 (s, 3H).

### 1-[2-Deoxy-3-O-acetyl-4-thio-β-D-ribofuranosyl]uracil (44)

Trifluoroacetic acid (200 µL) was added to a dichloromethane solution (2 mL) of compound 43 (420 mg, 058 mmol), and the mixture was stirred at room temperature. The reaction solution was diluted with ethyl acetate, washed with a saturated sodium hydrogencarbonate aqueous solution (x2) and with a saturated sodium chloride aqueous solution (x1), and dried over Na₂SO₄. After cotton plug filtration, the solvent was distilled off in vacuo. The residue was purified by silica gel chromatography (hexane: AcOEt = 1:2 -> AcOEt) to obtain compound 44 (30 mg, 32%) as a white foam.
FAB-LRMS m/z 287 (MH⁺)
¹H NMR (270 MHz, CDCl₃) δ: 9.25 (br s, 1H), 8.07 (d, 1H, J = 8.0), 6.43 (m, 1H), 5.79 (d, 1H, J = 8.0), 5.41 (m, 1H), 3.98 (m, 1H), 3.77 (m, 1H), 3.57 (m, 1H), 2.75 (br s, 1H), 2.50 (m, 1H), 2.35 (m, 1H), 2.09 (s, 3H).

### 4'-Thio-2'-deoxyuridine 5'-triphosphate (45)

Compound 44 (28 mg, 0.1 mmol) was treated with salicyl phosphorochloridite (30 mg, 0.15 mmol) as in the synthesis of compound 24 to obtain compound 45 (40 mg, 72%) as a white powder.
FAB-HRMS Calcd for C₉H₁₃N₂Na₃O₁₃P₃S (MH⁺): 550.9044. Found: 550.9023.
³¹P-NMR(108 MHz, D₂O) δ: -6.51 (d, J = 20 Hz), -10.42 (d, J = 20 Hz), -21.08 (t, J = 20 Hz).

### Example 14

### Synthesis of 4'-thio-2'-deoxy CTP (47), -ATP (49), and -GTP (51) (Fig. 6)

### 4'-Thio-2'-deoxycytidine 5'-triphosphate (47)

Compound 46 (39 mg, 0.1 mmol) was treated with salicyl phosphorochloridite (30 mg, 0.15 mmol) as in the synthesis of compound 24 to obtain compound 47 (36 mg, 65%) as a white powder.
FAB-HRMS Calcd for C₉H₁₄N₃Na₃O₁₂P₃S (MH⁺): 549.9204. Found: 549.9219.
³¹P-NMR(108 MHz, D₂O) δ: -6.18 (d, J = 20 Hz), -10.37 (d, J = 20 Hz), -21.21 (t, J = 20 Hz).

### 4'-Thio-2'-deoxyadenosine 5'-triphosphate (49)

Compound 48 (41 mg, 0.1 mmol) was treated with salicyl phosphorochloridite (30 mg, 0.15 mmol) as in the synthesis of compound 24 to obtain compound 49 (35 mg, 61%) as a white powder. FAB-HRMS Calcd for C₁₀H₁₄N₅Na₃O₁₁P₃S (MH⁺): 573.9316. Found: 549.9237.
³¹P-NMR(108 MHz, D₂O) δ:-5.54 (d, J = 20 Hz), -10.54 (d, J = 20 Hz), -20.64 (t, J = 20 Hz).

### 4'-Thio-2'-deoxyguanosine 5'-triphosphate (51)

Compound 50 (46 mg, 0.1 mmol) was treated with salicyl phosphorochloridite (30 mg, 0.15 mmol) as in the synthesis of compound 24 to obtain compound 51 (32 mg, 55%) as a white powder.
FAB-HRMS Calcd for C₁₀H₁₄N₅Na₃O₁₂P₃S (MH⁺): 589.9265. Found: 589.9239.
³¹P-NMR(108 MHz, D₂O) δ: -5.30 (d, J = 20 Hz), -10.68 (d, J = 20 Hz), -20.60 (t, J = 20 Hz).

### Example 15

### Incorporation of 4'-thio UTP into RNA chain with T7 RNA polymerase

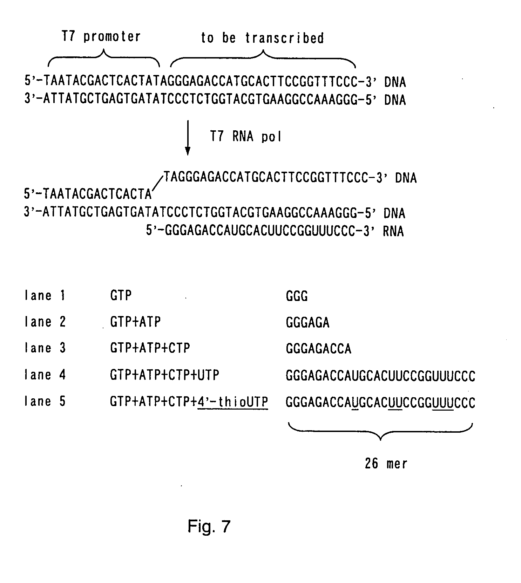

Incorporation experiment using T7 RNA polymerase was performed with 4'-thio UTP obtained in Example 9. In this experiment, double-stranded DNA containing T7 promoter sequence was used (Fig. 7). When all of the nucleotides ATP, GTP, CTP and UTP are present, a complementary 26mer RNA shown in the figure ought to be synthesized.

The reaction was conducted in 20 µL of a solution containing 40 mM Tris-HCl with PH 8.0, 8 mM MgCl₂, 2 mM spermidine, 5 mM DTT, 0.4 mM NTPs, 17 nM [γ-³²P]GTP, 2.0 µM template DNA and 100 U T7 RNA polymerase. NTPs were (1) GTP, (2) GTP + ATP, (3) GTP + ATP + CTP, (4) GTP + ATP + CTP + UTP, and (5) GTP + ATP + CTP + 4'-thio UTP, respectively. The reaction solution was incubated at 37°C for 3 hours, and the reaction was stopped. Subsequently, electrophoresis was performed with 20% modified polyacrylamide gel (30 x 40 x 0.05 cm, 1800 V, 1 hour, 1 x TEB), and analysed by autoradiography.

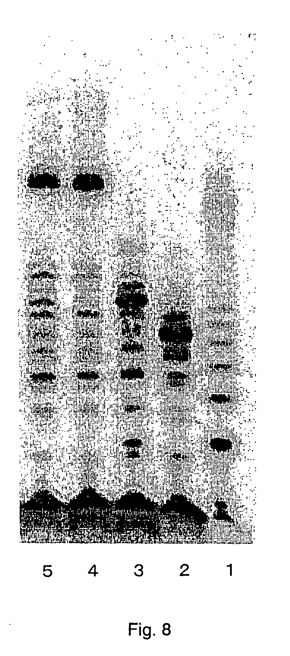

The results are shown in Fig. 8. In lane 1, bands corresponding to 2mer and 3mer of G and a rudder were observed. In lane 2, an expected band with chain elongation stopped at 6mer was observed, and a minor band elongated by one residue was observed. In lane 3, an expected band with chain elongation stopped at 9mer was observed, and a minor band elongated by one residue was observed. In lane 4, a band of 26mer full-length product was observed. In lane 5 where 4'-thio UTP was used instead of UTP, a band of a full-length product was observed at approximately the same amount as in lane 4. That is, it was demonstrated that 4'-thio UTP was also recognized by T7 RNA polymerase as a substrate and incorporated into RNA chain as with native UTP,

### Example 16

### Incorporation of 4'-thio UTP and 4'-thio CTP into RNA chain with T7 RNA polymerase

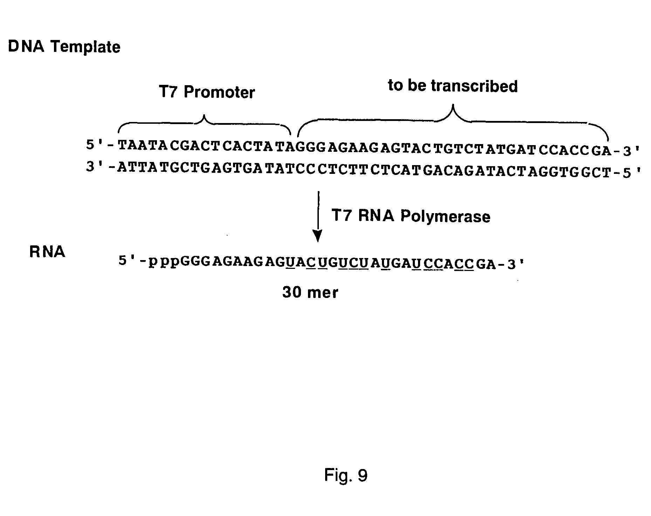

Incorporation experiment using T7 RNA polymerase was performed with 4'-thio UTP and 4'-thio CTP. In this experiment, 47mer double-stranded DNA containing T7 promoter sequence was used (Fig. 9). When all of the nucleotides ATP, GTP, CTP and UTP are present, a 30mer RNA shown in the figure ought to be synthesized.

The reaction was conducted in 20 µL of a solution containing 40 mM Tris-HCl with pH 8.0, 8 mM MgCl₂, 2 mM spermidine, 5 mM DTT, 0.4 mM NTPs, 17 nM [γ-³²P]GTP, 2.0 µM template DNA and 100 U T7 RNA polymerase. NTPs were (1) ATP, GTP, CTP and UTP, (2) using 4'-thio UTP instead of UTP, 3) using 4'-thio CTP instead of CTP, and 4) UTP and CTP were replaced with 4'-thio UTP and 4'-thio CTP. The reaction solution was incubated at 37°C for 3 hours, and the reaction was stopped. Subsequently, electrophoresis was performed with 20% modified polyacrylamide gel (30 x 40 x 0.05 cm, 1800 V, 1 hour, 1 x TEB), and analysed by autoradiography.

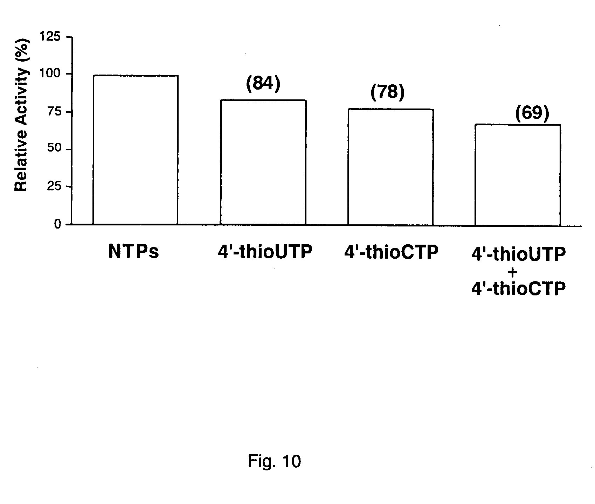

A 30mer RNA as a full-length product was observed in all of the experiment using 4'-thio UTP instead of UTP, the experiment using 4'-thio CTP instead of CTP and the experiment in which both NTPs were replaced with modified NTPs. A relative transcription efficiency is shown in Fig. 10 with a transcription efficiency of native NTPs being defined as 100. The results revealed that 4'-thio UTP and 4'-thio CTP are also recognized by T7 RNA polymerase as a substrate and incorporated into RNA chain efficiently.

### Example 17

### Synthesis of cDNA from RNA chain containing 4'-thio UTP and 4'-thio CTP

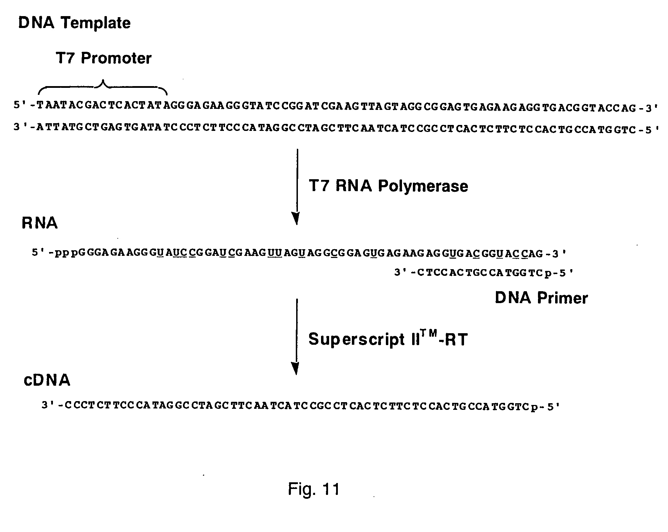

It was examined whether cDNA can be obtained using as a template 4'-thio RNA obtained by a transcription reaction in Example 16 and reverse transcriptase. To perform this experiment, RNA having a longer chain is required. A 76mer double-stranded DNA shown in Fig. 11 was used as a template. First, a transcription reaction with T7 RNA polymerase was conducted in the presence of GTP, ATP, 4'-thio UTP and 4'-thio CTP. The reaction was conducted under the same conditions as in Example 16, and a transcript 4'-thio RNA could be obtained efficiently. cDNA was synthesized by reverse transcription reaction using the resulting 59mer 4'-thio RNA as a template and reverse transcriptase.

The reaction was conducted by the following method. 4 µL (20 pmol) of a primer with a 5'-terminal radiolabeled and 6 µL (20 pmol) of 59mer RNA were mixed, and the following dNTP was added to each solution.
Lane 1: primer
Lane 2: no enzyme
Lane 3 and lane 7: dTTP
Lane 4 and lane 8: dTTP, dTCP
Lane 5 and lane 9: dTTP, dCTP, dATP
Lane 2, lane 6 and lane 10: dTTP, dCTP, dATP, dGTP

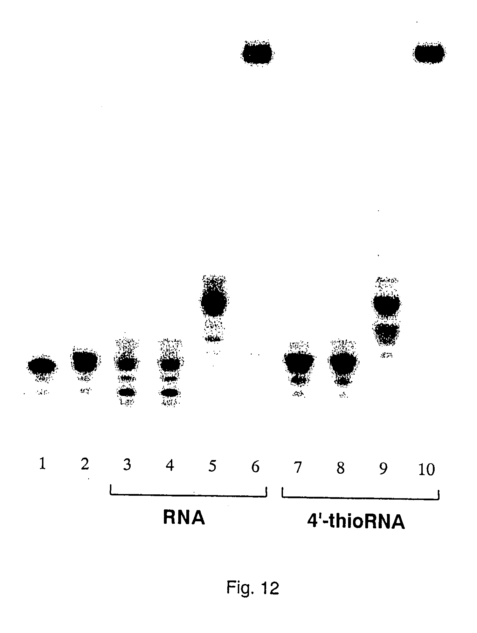

Each of the reaction solutions was incubated at 70°C for 15 minutes, and immediately incubated at 0°C for 1 minute or more. Subsequently, 2 µL of 0.1 M DTT, 4 µL of 5 X first strand buffer (250 mM Tris-HC1 (PH 8.3), 375 mM KCl and 15 mM MgCl₂), 1 µL of RNase Out and 1 µL of SuperScript™ II RNase⁻ reverse transcriptase were added to the solution. Except that, in lane 2, 1 µL of sterile water was added instead of SuperScript™ II RNase⁻ reverse transcriptase. The reaction solutions were mixed, incubated at 42°C for 50 minutes, then the reaction was stopped. Electrophoresis was performed with 12% modified polyacrylamide gel (30 x 30 x 0.05 cm, 1300 V, 1 hour, 1 x TBE), and analysed by autoradiography. The results are shown in Fig. 12.

It has been found that even when 4'-thio RNA was used as a template, the reverse transcription reaction proceeded, and cDNA can be synthesized at the same efficiency as in using native RNA as a template. The resulting cDNA was sequenced, and it has been confirmed that the sequence is correct.

### Example 18

### RNase A resistance of RNA chain containing 4'-thio UTP and 4'-thio CTP

An experiment of enzyme resistance of 59mer 4'-thio RNA obtained in Example 16 was performed using RNase A.

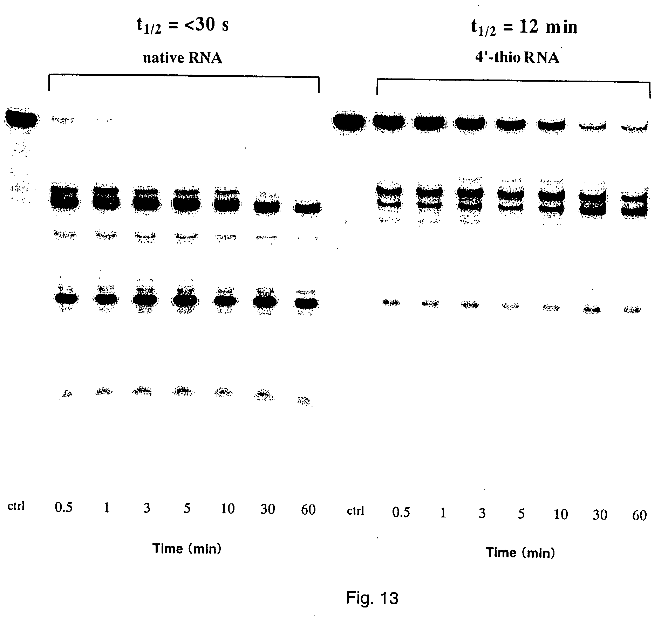

The reaction was conducted in 30 µL of a solution containing 10 mM Tris-HCl (PH 7.4), 5 mM EDTA (PH 7.5), 300 mM NaCl, 0.25 mM RNase A and 80 pmol native RNA or 4'-thio RNA. The reaction solution was incubated at 0°C. Samples were taken after 30 seconds, 1 minute, 3 minutes, 5 minutes, 10 minutes, 30 minutes and 60 minutes, and 4 µL aliquot of the reaction solution was mixed with 7 µL of a loading solution containing 10 M urea, 50 mM EDTA, XC (0.1%) and BPB (0.1%)to stop the reaction. Subsequently, electrophoresis was performed with 16% modified polyacrylamide gel (30 x 30 x 0.05 cm, 600 V, 1.5 hours, 1 x TBE), and analysed by autoradiography. The results are shown in Fig. 13.

4'-Thio RNA showed high resistance under such conditions that native RNA underwent enzymatic degradation by 90% or more for 30 seconds, and its half-life was 12 minutes. It was found that the residual amount of the full-length product after 1 hour with 4'-thio RNA is larger than the residual amount of the full-length product after 30 seconds with the native RNA, indicating that 4'-thio RNA is stable against RNase A by 100 times or more than native RNA.

## Claims

1. A compound of formula I: 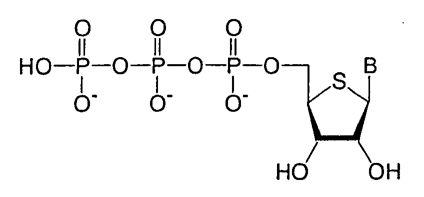 [wherein B is a nucleobase selected from the group consisting of adenine, guanine, cytosine, uracil and hypoxanthine].

2. A compound of formula II: 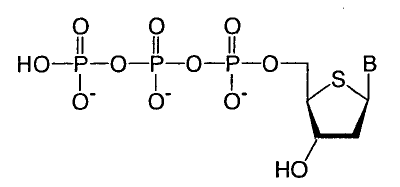 [wherein B' is a nucleobase selected from the group consisting of adenine, guanine, cytosine, thymine, uracil and hypoxanthine].

3. A method for synthesizing a compound of formula I: 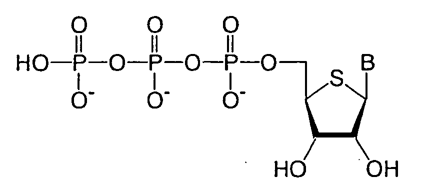 [wherein B is a nucleobase selected from the group consisting of adenine, guanine, cytosine, uracil and hypoxanthine], said method comprising reacting a compound of formula III: 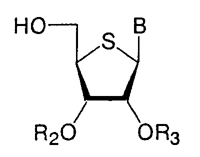 [wherein B is a nucleobase selected from the group consisting of adenine, guanine, cytosine, uracil and hypoxanthine, and each of R₂ and R₃ is, independently a protecting group of a hydroxyl group]
with a compound of formula IV: 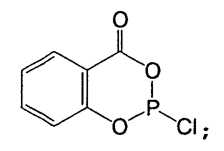 reacting the resulting intermediate with pyrophosphoric acid; and
conducting iodo-oxidation, hydrolysis and deprotection to obtain the compound of formula I.

4. A method for synthesizing a compound of formula II: 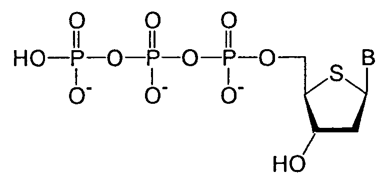 [wherein B is a nucleobase selected from the group consisting of adenine, guanine, cytosine, thymine, uracil and hypoxanthine],
said method comprising reacting a compound of formula V: 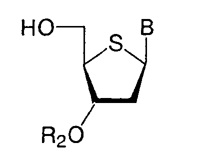 [wherein B is a nucleobase selected from the group consisting of adenine, guanine, cytosine, thymine, uracil and hypoxanthine, and R₂ is a protecting group of a hydroxyl group] with a compound of formula IV: 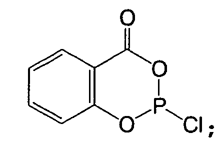 reacting the resulting intermediate with pyrophosphoric acid; and
conducting iodo-oxidation, hydrolysis and deprotection to obtain the compound of formula II.

5. A process for producing an oligonucleotide containing at least one nucleoside unit of formula VI: 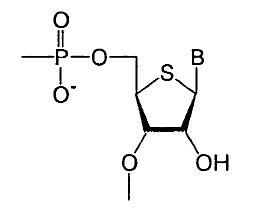 [wherein B is a nucleobase selected from the group consisting of adenine, guanine, cytosine, uracil and hypoxanthine] comprising conducting RNA chain elongation reaction with RNA synthetase in the presence of the compound of claim 1 or the compound produced by the method according to claim 3.

6. A process for producing an oligonucleotide containing at least one nucleotide unit of formula VII: 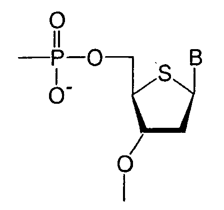 [wherein B is a nucleobase selected from the group consisting of adenine, guanine, cytosine, thymine, uracil and hypoxanthine]
comprising conducting DNA chain elongation reaction with DNA synthetase in the presence of the compound of claim 2 or the compound produced by the method according to claim 4.
